# DEMANDE DE BREVET EUROPEEN

(11) **EP 1 671 680 A1**
(43) Date de publication de la demande: **21.06.2006**
(21) Numéro de dépôt: 05292563.3
(22) Date de dépôt: 02.12.2005
(51) Int. Cl.: A61Q 19/04, A61K 8/42, A61K 8/35

(54) **Produit autobronzant à deux composants, dispositifs multicompartiments, procédé de coloration de la peau**

(30) Priorité: 20.12.2004 FR 0453080
(71) Demandeur: L'ORÉAL, 75008 Paris (FR)
(72) Inventeur: Candau, Didier, 91570 Bievres (FR)
(74) Mandataire: Miszputen, Laurent

(57) **Abrégé**

La présente invention se rapporte à un produit autobronzant pour la peau comprenant au moins deux composants (A) et (B) comprenant
- un premier composant (A) contenant dans un milieu cosmétiquement acceptable au moins un agent autobronzant et
- un deuxième composant (B) contenant dans un milieu cosmétiquement acceptable au moins une hydroxyalkylurée de formule (I) 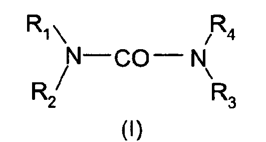
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.
; les composants (A) et (B) étant dans la même composition ou bien stockés séparément.

La présente invention concerne également divers dispositifs à plusieurs compartiments encore appelé « kit « ou « nécessaire de coloration de la peau, comportant
- un premier compartiment comprenant le composant (A) renfermant ledit agent autobronzant et
- un deuxième compartiment comprenant le composant (B) renfermant l'hydroxyalkylurée.

La présente invention concerne également l'utilisation d'au moins une hydroxyalkylurée de formule (I) dans un produit autobronzant pour la peau à au moins deux composants, dans le but d'améliorer la coloration dudit agent autobronzant.

## Description

La présente invention se rapporte à un produit autobronzant pour la peau comprenant au moins deux composants (A) et (B) comprenant
- un premier composant (A) contenant dans un milieu cosmétiquement acceptable au moins un agent autobronzant et
- un deuxième composant (B) contenant dans un milieu cosmétiquement acceptable au moins une hydroxyalkylurée de formule (I) ; les composants (A) et (B) faisant partie de la même composition ou bien étant stockés séparément.
   La présente invention concerne également divers dispositifs à plusieurs compartiments encore appelé « kit « ou « nécessaire de coloration de la peau, comportant
- un premier compartiment comprenant le composant (A) renfermant le ou les agents autobronzants et
- un deuxième compartiment comprenant le composant (B) renfermant l'hydroxyalkylurée.
   La présente invention se rapporte encore à un procédé de traitement cosmétique pour bronzer ou brunir artificiellement la peau utilisant cet agent autobronzant.
   Par "agent autobronzant" au sens de la présente demande, on entend un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV.
   De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire des réactions voire une altération de celle-ci, notamment dans le cas d'une peau sensible ou d'une peau continuellement exposée au rayonnement solaire : érythème, brûlure, perte d'élasticité, apparition de rides, vieillissement prématuré. Il est donc souhaitable de trouver une alternative au bronzage naturel qui soit compatible avec les exigences de telles peaux.
   La plupart des produits cosmétiques destinés au bronzage artificiel de la peau sont à base de dérivés carbonylés permettant, par interaction avec les acides aminés et/ou les peptides de la peau (due essentiellement à une réaction du type MAILLARD), la formation de produits colorés, parmi ceux-ci on compte des composés mono- ou polycarbonylés tels que par exemple l'isatine, l'alloxane, la ninhydrine, le glycéraldéhyde, l'aldéhyde mésotartrique, la glutaraldéhyde, l'érythrulose, la dihydroxyacétone (DHA).
   La DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.
   Un inconvénient de la DHA est la lenteur avec laquelle la coloration se développe : il faut en effet compter plusieurs heures (3 à 5 heures en général) pour que soit révélée la coloration. L'intensité de la coloration obtenue sur la peau et /ou sa tenue au cours du temps (notamment la résistance au lavage) et/ou la rapidité avec laquelle la coloration se développe, et/ou son homogénéité sont souvent jugées insuffisantes par les utilisateurs de compositions autobronzantes à base de DHA.
   Il existe donc une demande croissante de produits autobronzants agissant rapidement et conférant une coloration proche du bronzage naturel.
   De manière surprenante et avantageuse, la demanderesse a trouvé que l'on pouvait remédier aux divers inconvénients énoncés ci-dessus en utilisant un agent produit autobronzant pour la peau à au moins deux composants stockés séparément comprenant un premier composant (A) contenant au moins un agent autobronzant et un deuxième composant (B) contenant au moins une hydroxyalkylurée de formule (I). Un tel composé permet d'améliorer la coloration des compositions comprenant un agent autobronzant notamment au niveau de la rapidité du développement de la couleur après application, de l'intensité de la couleur, de l'homogénéité sur la peau et de la stabilité de la couleur obtenue.
   La présente invention concerne donc un produit autobronzant pour la peau à au moins deux composants stockés de manière séparée, caractérisé par le fait qu'il comprend au moins
- un premier composant (A) contenant dans un milieu cosmétiquement acceptable au moins un agent autobronzant et
- un deuxième composant (B) contenant dans un milieu cosmétiquement acceptable au moins une hydroxyalkylurée de formule (I) ; les composants (A) et (B) faisant partie de la même composition ou stockés séparément.
   La présente invention concerne également divers dispositifs à plusieurs compartiments encore appelé « kit « ou « nécessaire de coloration de la peau, comportant
- un premier compartiment comprenant le composant (A) renfermant le ou les agents autobronzants et
- un deuxième compartiment comprenant le composant (B) renfermant l'hydroxyalkylurée.

La présente invention concerne également un procédé de traitement cosmétique pour bronzer ou brunir artificiellement la peau caractérisé par le fait que l'on applique sur la peau le composant (A) et le composant (B) soit simultanément dans une même composition , soit de façon successive ou décalée dans le temps.

Conformément au procédé de l'invention, lorsque l'application se fait en deux temps le composant (A) peut être appliqué avant ou après l'application du composant (B).

La présente invention concerne également l'utilisation d'au moins une hydroxyalkylurée de formule (I) tel que définie ci-dessous dans un produit autobronzant pour la peau à au moins deux composants comprenant un premier composant (A) contenant au moins un agent autobronzant et un deuxième composant (B) contenant au moins une hydroxyalkylurée, dans le but d'améliorer la coloration dudit agent autobronzant.

D'autres caractéristiques, aspects et avantages de la présente invention apparaîtront à la lecture de la description détaillée qui va suivre.

Les hydroxyalkylurées conformes à l'invention sont choisies par celles répondant à la formule générale (I) : 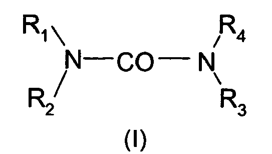 dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

Dans la formule (I), parmi les groupes alkyle, on peut notamment citer les groupes méthyle, éthyle, n-propyle, isopropyle, n-butyle, iso-butyle et tert-butyle.

Les composés de formule (I) préférés sont ceux ne renfermant qu'un seul groupe hydroxyalkyle, c'est-à-dire ceux pour lesquels R₁ est un groupe hydroxyalkyle et R₂, R₃ et R₄ représentent, indépendamment les uns des autres, un atome d'hydrogène ou un groupe alkyle en C₁-C₄. On préfère plus particulièrement les composés de formule (I) pour lesquels R₁ est un groupe hydroxyalkyle et R2, R3 et R4 représentent chacun un atome d'hydrogène.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle. Le groupe hydroxyéthyle est préféré.

Comme composés de formule (I) préférés, on peut citer la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée; la N-méthyl-N-(1,3,4,5,6-pentahydroxy-2-hexyl)- urée; la N-méthyl-N'-(1-hydroxy-2-méthyl-2-propyl)- urée ; la N-(1-hydroxy-2-méthyl-2-propyl)- urée; la N-(1,3-dihydroxy-2-propyl)- urée; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée; la N,N-Bis-(2-hydroxyéthyl)-N'-propyl- urée ; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée; la N-(1,3-dihydroxy-2-propyl)-N'-(2-hydroxyéthyl)- urée; la N,N-Bis-(2-hydroxyéthyl)-N',N'-dimethyl- urée ; la N,N,N',N'-tetrakis-(2-hydroxyéthyl)- urée ; et la N',N'-Bis-(2-hydroxyéthyl)-N',N'-bis-(2-hydroxypropyl)- urée.

Un composé particulièrement préféré pour une utilisation dans la présente invention est la N-(2-hydroxyéthyl)-urée, ci-après désignée par "hydroxyéthyl urée".

Les hydroxyalkylurées de formule (I) peuvent être préparées comme décrit dans la demande DE-27 03 185. Parmi celles-ci, l'hydroxyéthyl urée est en outre disponible dans le commerce, sous forme de mélange à 50% en poids dans l'eau, auprès de la société NATIONAL STARCH sous la dénomination commerciale Hydrovance®.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Lorsque les composants (A) et (B) sont dans la même composition, l'hydroxyalkylurée ou les hydroxyalkylurées conformes à l'invention sont de préférence présentes à des teneurs de 0,01 à 50% en poids et plus préférentiellement de 0,1 à 20 % et encore plus préférentiellement de 0,1 à 10% en poids par rapport au poids total de la composition contenant les composants (A) et (B).

Lorsque les composants (A) et (B) sont stockés séparément, l'hydroxyalkylurée ou les hydroxyalkylurées conformes à l'invention sont de préférence présentes à des teneurs de 0,01 à 50% en poids et plus préférentiellement de 0,1 à 20 % et encore plus préférentiellement de 0,1 à 10% en poids par rapport au poids total du composant (B).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Lorsque les composants (A) et (B) sont dans la même composition, le ou les agents autobronzants sont généralement présents dans des proportions allant de 0,1 à 15% en poids par rapport au poids total de la composition, et de préférence de 0,2 à 10% en poids et plus préférentiellement de 1 à 8 % en poids par rapport au poids total de la composition contenant les composants (A) et (B).

Lorsque les composants (A) et (B) sont stockés séparément, le ou les agents autobronzants sont généralement présents dans des proportions allant de 0,1 à 15% en poids par rapport au poids total de la composition, et de préférence de 0,2 à 10% en poids et plus préférentiellement de 1 à 8 % en poids par rapport au poids total du composant (A).

Lorsque l'autobronzant utilisé est la dihydroxyacétone (DHA), les composants (A) et (B) seront de préférence stockés séparément.

Les compositions de l'invention constituées soit par l'un et/ou l'autre des composants (A) et (B) ou par la composition contenant les deux composants (A) et (B) sont généralement adaptées à une application topique sur la peau et comprennent généralement un milieu physiologiquement acceptable, c'est-à-dire compatible avec la peau. Il s'agit d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition

Les compositions de l'invention peuvent se présenter sous toutes les formes appropriées pour une application topique, notamment sous forme de gels aqueux, sous forme d'émulsions obtenues par dispersion d'une phase grasse (appelée aussi phase huileuse) dans une phase aqueuse (H/E) ou inversement (E/H) ou d'émulsions multiples (par exemple E/H/E ou H/E/H ou H/H/E). Elles peuvent être plus ou moins fluides et avoir l'aspect d'une crème blanche ou colorée, d'une pommade, d'un lait, d'une lotion, d'un sérum, d'une pâte, de poudre, de bâtonnet solide et éventuellement être conditionnée en aérosol et se présenter sous forme de mousse ou de spray. Ces compositions sont préparées selon les méthodes usuelles.

Selon un mode particulier de réalisation de l'invention, les compositions de l'invention peuvent se présenter sous forme d'une émulsion et comporter alors au moins une phase huileuse. La proportion de la phase huileuse de l'émulsion peut aller de 1 à 80 % en poids, de préférence de 2 à 50 % en poids et mieux de 2 à 40 % en poids par rapport au poids total de la composition. Les corps gras de la phase huileuse, notamment les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant ,quand ils sont présents, le sont généralement, en une proportion allant de 0,1 à 30 % en poids, de préférence de 0,3 à 20 % en poids et mieux de 0,5 à 15 % en poids par rapport au poids de la composition. L'émulsion peut en outre, contenir des vésicules lipidiques en plus ou à la place des émulsionnants et/ou co-émulsionnants.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylés ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100 (nom CTFA : Glyceryl Stearate / PEG-100 Stearate) commercialisé sous la dénomination Arlacel 165 par la société Uniqema et sous la dénomination SIMULSOL 165 par la société SEPPIC ; ou le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition (A) ou (B), en utilisant des composés appropriés, pour stabiliser lesdites émulsions par exemple des polymères amphiphiles, des électrolytes.

Quand la composition de l'invention est sous forme d'émulsion, elle comporte au moins une phase huileuse qui contient au moins une huile, notamment une huile cosmétique. On entend par "huile" un corps gras liquide à la température ambiante (25°C).

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité ; les huiles de synthèse ; les huiles de silicone comme les polyméthylsiloxanes (PDMS) volatiles ou non à chaîne siliconée linéaire ou cyclique, liquides ou pâteux à température ambiante ; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les benzoates d'alcools gras en C₁₂-C₁₅ (Finsolv TN de FINETEX) ; les dérivés arylalkyl benzoates comme le 2-phenylethyl benzoate (X-Tend 226 de ISP) ; les huiles amidées comme le N-lauroylsarcosinate d'isopropyle (ELDEW SL-205 de Ajimoto) et leurs mélanges.

La composition de l'invention peut également contenir un ou plusieurs solvants organiques qui peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Les compositions conformes à la présente invention peuvent également comprendre des adjuvants cosmétiques classiques choisis parmi les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Comme épaississants hydrophiles, on peut citer les polymères carboxyvinyliques tels que les carbopols (carbomers) et les Pemulen (Copolymère acrylate/C10-C30-alkylacrylate) ; les dérivés cellulosiques tels que l'hydroxyéthylcellulose; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Comme conservateurs, on peut citer les esters de l'acide parahydroxybenzoïque encore appelés Parabens® (en particulier le méthyl parabène, l'éthyl parabène, le propyl parabène), le phénoxyéthanol, les libérateurs de formol comme par exemple l'imidazolidinyl urée ou la diazolidinyl urée, le digluconate de chlorhexidine, le benzoate de sodium, le caprylyl glycol, l'iodo propynyl butyl carbamate, le pentylène glycol, le bromure d'alkyl triméthylammonium tel que le bromure de myristyl-triméthylammonium (nom CTFA : bromure de Myrtrimonium), le bromure de dodécyl-triméthylammonium, le bromure d'hexadécyl-triméthylammonium, et leurs mélanges tel que le mélange vendu sous la dénomination Cetrimide® par la société FEF CHEMICALS.Le conservateur peut être présent dans la composition selon l'invention en une teneur allant de 0,001 à 10 % en poids, par rapport au poids total de la composition, notamment allant de 0,1 à 5 % en poids, et en particulier allant de 0,2 à 3 % en poids.

Les composants (A) et/ou (B) conformes à la présente invention peuvent également comprendre un ou plusieurs agents de coloration additionnels.

Au sens de la présente invention, on entendra par « agent de coloration de la peau », tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage.

Les agents de coloration additionnels peuvent également être choisis par exemple parmi d'autres extraits végétaux comme par exemple les extraits de sorgho obtenus à partir de la plante entière, des tiges, des graines ou des feuilles du genre Sorghum. Les espèces préférées du Sorghum sont choisies parmi le Sorghum bicolor, le Sorghum caudatum, le Sorghum nervosum, le Sorghum durra, le Sorghum vulgare et les Sorghum en association avec du Colletotrichum Graminicola comme ceux décrits dans la demande de brevet FR0200251.

Les agents de coloration additionnels peuvent également être des pigments d'oxyde de fer dont la taille moyenne des particules élémentaires est inférieure à 100 nm tels que ceux décrits dans la demande de brevet EP 966 953.

Les composants (A) et/ou (B) conformes à la présente invention conformes à la présente invention peuvent également comprendre des colorants permettant de colorer les compositions de l'invention pour faciliter le repérage de la zone de la peau à colorer et/ou pour modifier la couleur produite par l'agent autobronzant.

Ces colorants peuvent être choisis parmi les colorants directs synthétiques ou naturels.

Ces colorants peuvent être choisis par exemple parmi les colorants rouges ou oranges du type fluorane tels que ceux décrits dans la demande de brevet FR2840806. On peut citer par exemple les colorants suivants :
- le tetrabromofluoroscéine ou éosine connue sous le nom CTFA : CI 45380 ou Red 21
- la phloxine B connue sous le nom CTFA : CI 45410 ou Red 27
- la diiodofluorescéine connue sous le nom CTFA CI 45425 ou Orange 10 ;
- la dibromofluorescéine connue sous le nom CTFA : CI 45370 ou Orange 5.
- le sel de sodium de la tetrabromofluoroscéine connue sous le nom CTFA : CI 45380 (Na salt) ou Red 22
- le sel de sodium de la phloxine B connu sous le nom CTFA : CI 45410 (Na salt) ou Red 28
- le sel de sodium de la düodofluorescéine connu sous le nom CTFA : CI 45425 (Na salt) ou Orange 11 ;
- l'érythrosine connu sous le nom CTFA : CI 45430 ou Acid Red 51.
- la phloxine connu sous le nom CTFA : CI 45405 ou Acid Red 98.

Ces colorants peuvent être également choisis parmi les antraquinones , le caramel, le carmin, le noir de charbon, , les bleus azulènes, le methoxalène, le trioxalène, le guajazulène, le chamuzulène, le rose de bengale, la cosine 10B, la cyanosine, la daphinine.

Ces colorants peuvent être également choisis parmi les dérivés indoliques comme les monohydroxyindoles tels que décrits dans le brevet FR2651126 (ie : 4-, 5-, 6- ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole) ;

Les composants (A) et/ou (B) conformes à l'invention comporteront de préférence d'autres agents photoprotecteurs organiques ou inorganiques complémentaires actifs dans l'UVA et/ou l'UVB hydrosolubles ou liposolubles ou bien insolubles dans les solvants cosmétiques couramment utilisés.

Les agents photoprotecteurs organiques complémentaires sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs organiques complémentaires, on peut citer ceux désignés ci-dessous sous leur nom INCI :

Dérivés de l'acide para-aminobenzoique :
PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

Dérivés du dibenzovlméthane :
Butyl Methoxydibenzoylmethane vendu notamment sous le nom commercial « PARSOL 1789 » par HOFFMANN LAROCHE,
Isopropyl Dibenzoylmethane,

Dérivés salicyliques :
Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

Dérivés cinnamiques :
Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

Dérivés de β,β-diphénvlacrylate :
Octocrylene vendu notamment sous le nom commercial « UVINUL N539 » par BASF,
Etocrylene, vendu notamment sous le nom commercial « UVINUL N35 » par BASF,

Dérivés de la benzophénone :
Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

Dérivés du benzylidène camphre :
3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

Dérivés du phenvl benzimidazole :
Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

Dérivés du phenyl benzotriazole :
Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés de triazine :
Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris(4'-amino benzalmalonate de dinéopentyle)-s-triazine
la 2,4,6-tris-(4'-amino benzalmalonate de diisobutyle)-s- triazine.

Dérivés anthraniliaues :
Menthyl anthranilate vendu sous le nom commercial commercial « NEO HELIOPAN MA » par Haarmann et REIMER,

Dérivés d'imidazolines :
Ethylhexyl Dimethoxybenzylidene Dioxoimidazoline Propionate,

Dérivés du benzalmalonate :
Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

Dérivés de 4,4-diarvlbutadiène :
- 1,1-dicarboxy (2,2'-diméthyl-propyl)-4,4-diphénylbutadiène

Dérivés de benzoxazole :
2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les agents photoprotecteurs organiques complémentaires préférentiels sont choisis parmi
Ethylhexyl Methoxycinnamate
Homosalate
Ethylhexyl Salicylate,
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine.
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les agents photoprotecteurs inorganiques sont choisis parmi les pigments (taille moyenne des particules primaires : généralement entre 5 nm et 100 nm, de préférence entre 10 nm et 50 nm) d'oxydes métalliques enrobés ou non comme par exemple des pigments d'oxyde de titane (amorphe ou cristallisé sous forme rutile et/ou anatase), de fer, de zinc, de zirconium ou de cérium et leurs mélanges. Des agents d'enrobage classiques sont par ailleurs l'alumine et/ou le stéarate d'aluminium. De tels pigments d'oxydes métalliques, enrobés ou non enrobés, sont en particulier décrits dans les demandes de brevets EP 518 772 et EP 518 773.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Lorsque les composants (A) et (B) sont dans une même composition ou appliqués sous forme de mélange, le pH de la composition ou dudit mélange varie de préférence de 3,5 à 6,5 et plus préférentiellement de 4 à 6.

Bien entendu, l'homme de l'art veillera à choisir le ou les éventuels composés complémentaires cités ci-dessus et/ou leurs quantités de manière telle que les propriétés avantageuses attachées intrinsèquement aux deux composants (A) et (B) constituant le produit autobronzant conforme à l'invention ne soient pas, ou substantiellement pas, altérées par la ou les adjonctions envisagées. L'homme de l'art veillera également à incorporer l'additif ou les additifs cités ci-dessus de manière appropriée dans l'un des deux composants (A) et (B) lorsque ceux-ci sont stockés séparément.

Lorsque les composants (A) et/ou (B) sont stockés séparément, ils sont conditionnés dans un dispositif à plusieurs compartiments encore appelé « kit» ou « nécessaire de coloration de la peau », comportant
- un premier compartiment comprenant le composant (A) renfermant le ou les agents autobronzants et
- un deuxième compartiment comprenant le composant (B) renfermant l'hydroxyalkylurée.

Selon une première variante de dispositif conforme à l'invention, les composants (A) et (B) peuvent être conditionnés de manière indépendante chacun dans un récipient délimitant au moins un compartiment, ledit récipient étant fermé par un élément de fermeture.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Selon une autre variante, les composants respectivement (A) et (B) sont conditionnés dans deux récipients accolés de manière à être solidaire l'un de l'autre.

Par exemple, elles sont contenues dans deux récipients retenus l'un avec l'autre via un emballage externe. En particulier, chaque récipient est muni d'un organe de distribution, par exemple une pompe ou une valve. De préférence cet organe de distribution est à actionnement manuel. La pompe peut également être sans reprise d'air dans le cas où la composition doit être préservée de tout contact avec l'extérieur, pendant son temps de stockage.

Selon un premier mode de réalisation, l'actionnement de ces organes de distribution conduit les compositions dans une chambre de mélange de l'ensemble, de manière à assurer un mélange préalable à leur distribution hors de cet ensemble formé par ces deux récipients accolés

Alternativement, selon un premier mode de réalisation, l'actionnement de ces organes de distribution conduit les compositions dans une chambre de mélange de l'ensemble, de manière à assurer un mélange préalable à leur distribution hors de cet ensemble formé par ces deux récipients accolés.

Alternativement, selon un deuxième mode de réalisation, l'actionnement de ces organes de distribution conduit à une sortie des compositions sans mélange préalable.

Un mode particulier de l'invention est un dispositif tel que décrit dans la demande de brevet EP1270444. Il s'agit d'un dispositif pour la distribution simultanée des deux composants (A) et (B), conditionnés séparément dans des premier et second sachets à parois souples, ledit dispositif comprenant :
i) des moyens pour maintenir fixement les deux sachets en position superposée de sorte que des orifices de sortie respectifs desdits sachets soient disposés au voisinage l'un de l'autre ; et
ii) des moyens, mobiles par rapport aux moyens de fixation, et aptes à pressuriser les deux sachets de manière à en forcer la sortie de leur contenu au travers de leurs orifices de sortie respectifs selon un rapport prédéterminé.

Un autre mode particulier de l'invention est un dispositif tel que décrit dans la demande de brevet EP1300344. Il s'agit d'un dispositif pour la distribution conjointe des deux composants (A) et (B) conditionnés séparément, comprenant :
a) un premier récipient à parois souples contenant le composant (A);
b) un second récipient à parois souples, extérieur au premier, et contenant le composant (B);
c) une platine sur laquelle sont montés les premier et second récipients ;
d) un organe de distribution, mobile par rapport à la platine, et apte à faire passer le dispositif, de manière irréversible, d'une première configuration dans laquelle les premier et second récipients sont isolés d'au moins un orifice de distribution formé par ledit organe de distribution, à une seconde configuration dans laquelle les premier et second récipients sont en communication avec ledit (ou lesdits) orifice(s) de distribution.

Ainsi, dans la seconde configuration du dispositif, en réponse à une pression exercée sur les parois souples des premier et second récipients, lesquels sont disposés de manière adjacente l'un par rapport à l'autre, les premier et second produits peuvent être distribués conjointement.

La sortie conjointe des deux produits en vue de la réalisation d'une composition, notamment cosmétique, peut se faire via un orifice de distribution unique, en amont duquel peut se trouver une zone de mélange dans laquelle les deux produits sont mis en contact avant d'être distribués sous forme mélangée.

Un autre mode particulier de dispositif de l'invention est une double pompe comprenant
a) deux réservoirs, de préférence logés dans un même boîtier externe comprenant respectivement le composant (A) ou le composant (B), de préférence logés dans un même boîtier externe ;
b) deux pompes, chacune montée sur un réservoir,
c) un seul bouton poussoir, mobile axialement le long d'un axe d'allongement principal du boîtier externe, et permettant d'actionner l'ouverture simultanée des deux pompes ; ces deux pompes étant par ailleurs chacune reliée par un canal de sortie débouchant sur au moins un orifice de distribution.

Ledit orifice de distribution peut être unique et commun aux deux réservoirs. Dans ce cas les produits sortent sous forme mélangée.

Selon une autre variante, chaque canal de sortie est associé à un orifice de distribution qui lui est propre. Les produits contenus dans ces réservoirs sont donc distribués hors du dispositif, sans jamais avoir été mis en contact au préalable à l'intérieur du dispositif. Ce type de variante est notamment décrite dans les brevets FR2789371 ; US5, 224,627; W097/05040

Un autre mode particulier de dispositif de l'invention est un double aérosol comprenant
a) deux réservoirs, de préférence logés dans un même boîtier externe comprenant au moins un agent propulseur et respectivement le composant (A) ou le composant (B)
b) deux valves aérosols, chacune montée sur un réservoir,
c) un seul bouton poussoir, mobile axialement le long d'un axe d'allongement principal du boîtier externe, et permettant d'actionner l'ouverture simultanée des deux pompes ; ces deux valves étant par ailleurs chacune reliée par un canal de par un canal de sortie débouchant sur au moins un orifice de distribution.

Ledit orifice de distribution peut être unique et commun aux deux réservoirs. Dans ce cas les produits sortent sous forme mélangée.

Selon une autre variante, chaque canal de sortie est associé à un orifice de distribution qui lui est propre. Les produits contenus dans ces réservoirs sont alors distribués hors du dispositif, sans jamais avoir été mis en contact au préalable à l'intérieur du dispositif.

Bien entendu, en fonction du dispositif utilisé, l'homme de l'art veillera , pour chacun des composants (A) et (B), à choisir la viscosité et les propriétés rhéologiques adéquates pour permettre la bonne sortie des produits hors dudit dispositif.

L'invention va maintenant être décrite en référence aux exemples suivants donnés à titre illustratif et non limitatif. Dans ces exemples, sauf indication contraire, les quantités sont exprimées en pourcentages pondéraux.

### Exemple 1

On a réalisé le produit autobronzant à deux composants (A) et (B) suivants ; les quantités sont indiquées en pourcentages en poids :

| **Phase** | **Composition A** | **Quantités** |
|---|---|---|
| **1** | Polydiméthylsiloxane | 1,0 |
| | Conservateurs | 1,0 |
| | Mélange monostéarate de glycéryle/stéarate-PEG (100 OE) | 2,0 |
| | Tartrate de dialkyle (C₁₂/C₁₃) | 1,0 |
| | Mélange d'arachyl polyglycoside et d'alcools arachidique, béhénique (15/85) | 3,0 |
| | Benzoate d'alcools en C₁₂/C₁₅ | 6,0 |
| **2** | Copolymère acrylamide/acrylamido 2-méthyl sulfonate de sodium en émulsion inverse | 1,75 |
| **3** | Cyclopentadimethylsiloxane | 3,0 |
| **4** | Dihydroxyacétone | 5,0 |
| **5** | Glycérine | 5,0 |
| | EDTA | 0,1 |
| | Eau désionisée | qsp 100 |

| **Phase** | **Composition B** | **Quantités** |
|---|---|---|
| **1** | Polydiméthylsiloxane | 1,0 |
| | Conservateurs | 1,0 |
| | Mélange monostéarate de glycéryle/stéarate-PEG (100 OE) | 2,0 |
| | Tartrate de dialkyle (C₁₂/C₁₃) | 1,0 |
| | Mélange d'arachyl polyglycoside et d'alcools arachidique, béhénique (15/85) | 3,0 |
| | Benzoate d'alcools en C₁₂/C₁₅ | 6,0 |
| **2** | Copolymère acrylamide/acrylamido 2-méthyl sulfonate de sodium en émulsion inverse | 1,75 |
| **3** | Cyclopentadimethylsiloxane | 3,0 |
| | Hydroxyéthylurée | 5,0 |
| **5** | Glycérine | 5,0 |
| | EDTA | 0,1 |
| | Eau désionisée | qsp 100 |

### Mode opératoire

On pèse la phase 1 et la phase 5 séparément et on porte au bain marie vers 80°C. On met la phase 1 sous agitation au Moritz et on verse la phase 5. On laisse agiter 10 minutes sous agitation moyenne. On ajoute vers 60°C la phase 2 et on agite jusqu'à homogénéisation. On incorpore la phase 3, on homogénéise puis on ajoute pour la composition A la phase 4 avec la DHA. On homogénéise puis on arrête l'agitation.

### Exemple 2

On a réalisé le produit autobronzant à deux composants (A) et (B) suivants ; les quantités sont indiquées en pourcentages en poids :

| **Phase** | **Composition A** | **Quantités** |
|---|---|---|
| **1** | Polydiméthylsiloxane | 1,0 |
| | Conservateurs | 1,0 |
| | Mélange monostéarate de glycéryle/stéarate-PEG (100 OE) | 2,0 |
| | Tartrate de dialkyle (C₁₂/C₁₃) | 1,0 |
| | Mélange d'arachyl polyglycoside et d'alcools arachidique, béhénique (15/85) | 3,0 |
| | Benzoate d'alcools en C₁₂/C₁₅ | 6,0 |
| **2** | Copolymère acrylamide/acrylamido 2-méthyl sulfonate de sodium en émulsion inverse | 1,75 |
| **3** | Cyclopentadimethylsiloxane | 3,0 |
| **4** | Dihydroxyacétone | 5,0 |
| **5** | Glycérine | 5,0 |
| | EDTA | 0,1 |
| | Eau désionisée | qsp 100 |

| **Phase** | **Composition B** | **Quantités** |
|---|---|---|
| **1** | Polydiméthylsiloxane | 1,0 |
| | Conservateurs | 1,0 |
| | Mélange monostéarate de glycéryle/stéarate-PEG (100 OE) | 2,0 |
| | Tartrate de dialkyle (C₁₂/C₁₃) | 1,0 |
| | Mélange d'arachyl polyglycoside et d'alcools arachidique, béhénique (15/85) | 3,0 |
| | Benzoate d'alcools en C₁₂/C₁₅ | 6,0 |
| **2** | Copolymère acrylamide/acrylamido 2-méthyl sulfonate de sodium en émulsion inverse | 1,75 |
| **3** | Cyclopentadimethylsiloxane Hydroxyéthylurée | 10,0 |
| **5** | Glycérine | 5,0 |
| | EDTA | 0,1 |
| | Eau désionisée | qsp 100 |

### Mode opératoire

On pèse la phase 1 et la phase 5 séparément et on porte au bain marie vers 80°C. On met la phase 1 sous agitation au Moritz et on verse la phase 5. On laisse agiter 10 minutes sous agitation moyenne. On ajoute vers 60°C la phase 2 et on agite jusqu'à homogénéisation. On incorpore la phase 3, on homogénéise puis on ajoute pour la composition A la phase 4 avec la DHA. On homogénéise puis on arrête l'agitation.

## Revendications

1. Produit autobronzant pour la peau à au moins deux composants, **caractérisé par le fait qu'**il comprend au moins
- un premier composant (A) contenant dans un milieu cosmétiquement acceptable au moins un agent autobronzant et
- un deuxième composant (B) contenant dans un milieu cosmétiquement acceptable au moins une hydroxyalkylurée de formule (I) 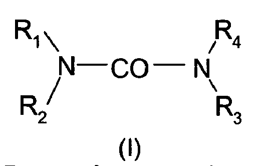
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.
; les composants (A) et (B) étant dans la même composition ou bien stockés séparément.

2. Produit selon la revendication 1, **caractérisée en ce que** R₁ est un groupe hydroxyalkyle et R₂, R₃ et R₄ représentent, indépendamment les uns des autres, un atome d'hydrogène ou un groupe alkyle en C₁-C₄.

3. Produit selon la revendication 2, **caractérisée en ce que** R₁ est un groupe hydroxyalkyle et R₂, R₃ et R₄ représentent chacun un atome d'hydrogène.

4. Composition selon l'une quelconque des revendications 1 à 3, **caractérisée en ce que** le composé de formule (I) est choisi parmi la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)-urée ; la N-méthyl-N-(1,3,4,5,6-pentahydroxy-2-hexyl)-urée; la N-méthyl-N'-(1-hydroxy-2-méthyl-2-propyl)- urée ; la N-(1-hydroxy-2-méthyl-2-propyl)- urée ; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée; la N,N-Bis-(2-hydroxyéthyl)-N'-propyl- urée ; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée ; la N-(1,3-dihydroxy-2-propyl)-N'-(2-hydroxyéthyl)- urée ; la N,N-Bis-(2-hydroxyéthyl)-N',N'-dimethyl- urée ; la N,N,N',N'-tetrakis-(2-hydroxyéthyl)- urée ; la N',N'-Bis-(2-hydroxyéthyl)-N',N'-bis-(2-hydroxypropyl)- urée.

5. Produit selon l'une quelconque des revendications 1 à 4, **caractérisée en ce que** l'hydroxyalkyl urée est la N-(2-hydroxyéthyl)-urée.

6. Produit selon l'une quelconque des revendications 1 à 5, où les composants (A) et (B) sont dans la même composition et où le ou les hydroxyalkylurées sont présentes à des teneurs de 0,01 à 50% en poids et plus préférentiellement de 0,1 à 20 % et encore plus préférentiellement de 0,1 à 10% en poids par rapport au poids total de la composition contenant les deux composants (A) et (B).

7. Produit selon l'une quelconque des revendications 1 à 5 où les composants (A) et (B) sont stockés séparément et où le ou les hydroxyalkylurées sont présentes à des teneurs de 0,01 à 50% en poids et plus préférentiellement de 0,1 à 20 % et encore plus préférentiellement de 0,1 à 10% en poids par rapport au poids total du composé (B).

8. Produit selon l'une quelconque des revendications 1 à 6, où l'agent autobronzant est un composé mono ou polycarbonylé.

9. Produit selon la revendication 8, telle que l'agent autobronzant est choisi dans le groupe formé par l'isatine, l'alloxane, la ninhydrine, le glycéraldéhyde, l'aldéhyde mésotartrique, la glutaraldéhyde, l'érythrulose, les dérivés de pirazoline -4,5-dione, la dihydroxyacétone (DHA), les dérivés de 4,4-dihydroxypirazoline-5-diones.

10. Produit selon la revendication 9, où l'agent autobronzant est la DHA.

11. Produit selon l'une quelconque des revendications 1 à 9, où les composants (A) et (B) sont dans la même composition et où le ou les agents autobronzants sont présents dans des proportions allant de 0,1 à 15% en poids par rapport au poids total de la composition, et de préférence de 0,2 à 10% en poids et plus préférentiellement de 1 à 8 % en poids par rapport au poids total de la composition contenant les composants (A) et (B).

12. Produit selon l'une quelconque des revendications 1 à 9, où les composants (A) et (B) sont stockés séparément et où le ou les agents autobronzants sont présents dans des proportions allant de 0,1 à 15% en poids par rapport au poids total de la composition, et de préférence de 0,2 à 10% en poids et plus préférentiellement de 1 à 8 % en poids par rapport au poids total du composant (A).

13. Produit selon l'une quelconque des revendications 1 à 9, où l'autobronzant utilisé est la dihydroxyacétone (DHA) et les composants (A) et (B) sont stockés séparément.

14. Produit selon l'une quelconque des revendications 1 à 13, où le composant (A) et/ou (B) comprend en plus un ou plusieurs agents de coloration additionnels.

15. Produit selon l'une quelconque des revendications 1 à 14, où le composant (A) et/ou (B) comprend en plus un ou plusieurs colorants directs synthétiques ou naturels.

16. Produit selon l'une quelconque des revendications 1 à 15, où le composant (A) et/ou (B) comprend en plus un ou plusieurs colorants indoliques.

17. Produit selon l'une quelconque des revendications 1 à 16, **caractérisée par le fait qu'**elle comporte en plus en outre au moins un agent photoprotecteur organique et/ou au moins un agent photoprotecteur inorganique actifs dans l'UVA et/ou dans l'UVB.

18. Produit selon l'une quelconque des revendications 1 à 17, **caractérisée par le fait que** la composition contenant les deux composants (A) et (B) ou le mélange des composants (A) et (B) a un pH de 3,5 à 6,5 et plus préférentiellement de 4 à 6.

19. Procédé de traitement cosmétique pour bronzer ou brunir artificiellement la peau **caractérisé par le fait que** l'on applique sur la peau le composant (A) et le composant (B) tels que définis dans les revendications précédentes soit simultanément dans une même composition, soit de façon successive ou décalée dans le temps.

20. Utilisation d'au moins une hydroxyalkylurée de formule (I) tel que définie dans les revendications précédentes dans un produit autobronzant pour la peau à au moins deux composants comprenant un premier composant (A) contenant au moins un agent autobronzant et un deuxième composant (B) contenant au moins une hydroxyalkylurée dans le but d'améliorer la coloration dudit agent autobronzant.

21. Dispositif à plusieurs compartiments pour la coloration de la peau, comportant
- un premier compartiment comprenant le composant (A) tel que défini dans l'une quelconque des revendications précédentes.
- un deuxième compartiment comprenant le composant (B) tel que défini dans l'une quelconque des revendications précédentes.

22. Dispositif selon la revendication 21, où les composants (A) et (B) sont conditionnés de manière indépendante chacun dans un récipient délimitant au moins un compartiment, ledit récipient étant fermé par un élément de fermeture.

23. Dispositif selon la revendication 21, où les composants (A) et (B) sont conditionnés dans deux récipients accolés de manière à être solidaire l'un de l'autre.

24. Dispositif selon la revendication 23, où les composants (A) et (B) sont contenus dans deux récipients retenus l'un avec l'autre via un emballage externe.

25. Dispositif selon la revendication 24 où chaque récipient est muni d'un organe de distribution, de préférence à actionnement manuel.

26. Dispositif selon la revendication 25 où l'organe de distribution est une pompe ou une valve aérosol.

27. Dispositif selon l'une quelconque des revendications 21 à 26, où l'actionnement de des organes de distribution conduit les composants (A) et (B) dans une chambre de mélange de l'ensemble, de manière à assurer un mélange préalable à leur distribution hors de cet ensemble formé par ces deux récipients accolés

28. Dispositif selon l'une quelconque des revendications 21 à 26, où l'actionnement de des organes de distribution conduit les composants (A) et (B) à une sortie des desdits composants sans mélange préalable.

29. Dispositif selon la revendication 27, **caractérisé par le fait que** les deux composants
(A) et (B) sont conditionnés séparément dans des premier et second sachets à parois souples, ledit dispositif comprenant :
i) des moyens pour maintenir fixement les deux sachets en position superposée de sorte que des orifices de sortie respectifs desdits sachets soient disposés au voisinage l'un de l'autre ; et
ii) des moyens, mobiles par rapport aux moyens de fixation, et aptes à pressuriser les deux sachets de manière à en forcer la sortie de leur contenu au travers de leurs orifices de sortie respectifs selon un rapport prédéterminé.

30. Dispositif selon la revendication 27, **caractérisé par le fait qu'**il comprend :
a) un premier récipient à parois souples contenant le composant (A);
b) un second récipient à parois souples, extérieur au premier, et contenant le composant (B);
c) une platine sur laquelle sont montés les premier et second récipients ;
d) un organe de distribution, mobile par rapport à la platine, et apte à faire passer le dispositif, de manière irréversible, d'une première configuration dans laquelle les premier et second récipients sont isolés d'au moins un orifice de distribution formé par ledit organe de distribution, à une seconde configuration dans laquelle les premier et second récipients sont en communication avec ledit (ou lesdits) orifice(s) de distribution.

31. Dispositif selon l'une quelconque des revendications 21 à 26, **caractérisé en ce qu'**il consiste en une double pompe comprenant
a) deux réservoirs, de préférence logés dans un même boîtier externe comprenant respectivement le composant (A) ou le composant (B), de préférence logés dans un même boitier externe ;
b) deux pompes, chacune montée sur un réservoir,
c) un seul bouton poussoir, mobile axialement le long d'un axe d'allongement principal du boîtier externe, et permettant d'actionner l'ouverture simultanée des deux pompes ; ces deux pompes étant par ailleurs chacune reliée par un canal de sortie à au moins un orifice de distribution.

32. Dispositif selon l'une quelconque des revendications 21 à 26, **caractérisé en ce qu'**il consiste en un double aérosol comprenant
a) deux réservoirs, de préférence logés dans un même boîtier externe comprenant au moins un agent propulseur et respectivement le composant (A) ou le composant (B)
b) deux valves aérosols, chacune montée sur un réservoir,
c) un seul bouton poussoir, mobile axialement le long d'un axe d'allongement principal du boîtier externe, et permettant d'actionner l'ouverture simultanée des deux pompes ; ces deux valves étant par ailleurs chacune reliée par un canal de sortie à au moins un orifice de distribution.
